# EUROPEAN PATENT APPLICATION

(11) **EP 4 555 971 A1**
(43) Date of publication of application: **21.05.2025**
(21) Application number: 23840006.3
(22) Date of filing: 13.07.2023
(51) Int. Cl.: A61C 9/00, G06F 3/0484, G06F 3/0481, A61C 5/00, A61B 5/00, G06T 1/00, G06T 17/00, G06T 19/20, G16H 30/00

(54) **IMAGE PROCESSING METHOD, ELECTRONIC DEVICE AND COMPUTER-READABLE STORAGE MEDIUM**

(30) Priority: 13.07.2022 KR 20220086432; 12.07.2023 KR 20230090642
(71) Applicant: Medit Corp., Seoul 07207 (KR)
(72) Inventor: JEON, Ha Ram, Seoul 07207 (KR)
(74) Representative: Studio Torta S.p.A.
(86) International application number: PCT/KR2023/010064
(87) International publication number: WO 2024/014915

(57) **Abstract**

A method for processing an image, an electronic apparatus performing the same, and a computer readable storage medium storing the same may be provided. The method for processing an image includes receiving at least one intraoral image, aligning the at least one intraoral image to a occlusal plane, setting an inner surface of a splint based on the at least one aligned intraoral image, setting an outline of the splint based on the at least one intraoral image, setting an outer surface of the splint based on the outline, and displaying the at least one intraoral image, to which the splint is applied, generated based on the outer surface.

## Description

### [Technical Field]

The present disclosure relates to a method for processing an image, an electronic apparatus, and a computer readable storage medium.

### [Background Art]

In cases where the temporomandibular joint is fatigued and temporomandibular joint disorder is caused due to improper occlusion, a splint, an orthodontic device, may be used to induce a stable central relation regardless of the malocclusion.

When designing the splint, an outer surface of the splint should be set while considering a contact with a molar. However, in general, when designing the outer surface of the splint by processing an intraoral image, the contact with the molar may not be created or an additional design process is required even if the contact with the molar is created.

In the additional design process, there are a case where the outer surface of the splint is curved according to the shape of the tooth and a plurality of smoothing operations should be repeated even after the outer surface is formed, a case where unintended protrusions are created in the outer surface, a case where protrusions should be corrected manually, etc.

In addition, in the repetitive splint manufacturing process, there is a problem that causes fatigue by allowing users and patients to repeatedly perform the same operations for designing the outer surface of the splint.

### [Disclosure]

### [Technical Problem]

The present disclosure attempts to increase efficiency of splint manufacturing by minimizing an additional design process when setting an outer surface of a splint.

The present disclosure attempts to reduce fatigue in splint manufacturing by providing a splint outer surface design simulation to facilitate a process of users and patients performing an outer surface design.

### [Technical Solution]

According to an exemplary embodiment, a method for processing an image includes receiving at least one intraoral image, aligning the at least one intraoral image to a occlusal plane, setting an inner surface of a splint based on the at least one aligned intraoral image, setting an outline of the splint based on the at least one intraoral image, setting an outer surface of the splint based on the outline, and displaying the at least one intraoral image, to which the splint is applied, generated based on the outer surface.

According to another exemplary embodiment, an electronic apparatus includes: a user interface device; a processor; and a memory storing instructions executable by the processor, in which the processor executes the instructions to: receive at least one intraoral image; align the at least one intraoral image to a occlusal plane; set an inner surface of a splint based on the at least one aligned intraoral image; set an outline of the splint based on the at least one intraoral image; set an outer surface of the splint based on the outline; and display the at least one intraoral image, to which the splint is applied, generated based on the outer surface.

According to still another exemplary embodiment, there is provided a computer readable storage medium including computer readable instructions, in which the instructions cause the computer to: receive at least one intraoral image; align the at least one intraoral image to a occlusal plane; set an inner surface of a splint based on the at least one aligned intraoral image; set an outline of the splint based on the at least one intraoral image; set an outer surface of the splint based on the outline; and display the at least one intraoral image, to which the splint is applied, generated based on the outer surface.

### [Advantageous Effects]

According to the disclosed exemplary embodiments, it is possible to reduce the additional design processes when designing the outer surface of the splint.

### [Description of the Drawings]

FIG. 1 is a diagram for describing a system for processing an image including an electronic apparatus according to an exemplary embodiment.
FIG. 2 is a block diagram illustrating a configuration of an electronic apparatus according to an exemplary embodiment.
FIG. 3 is a flowchart illustrating a method for processing an image of an electronic apparatus according to an exemplary embodiment.
FIGS. 4 and 5 are diagrams for describing scan data, maxillary scan data, and mandibular scan data according to an exemplary embodiment.
FIG. 6 is a flowchart illustrating a method for processing an image of an electronic apparatus according to an exemplary embodiment.
FIG. 7 is a diagram for describing a step of generating a crop image according to an exemplary embodiment.
FIG. 8 is a diagram for describing a step of generating a smoothing image according to an exemplary embodiment.
FIGS. 9 and 10 are diagrams for describing a step of generating a flattened image according to an exemplary embodiment.
FIG. 11 is a diagram for describing a step of generating an offset image according to an exemplary embodiment.
FIGS. 12 and 13 are diagrams for describing a step of setting a morphing direction according to an exemplary embodiment.
FIGS. 14 to 17 are diagrams for describing a morphing step according to an exemplary embodiment.
FIG. 18 is a diagram for describing a step of adjusting an occlusal thickness according to an exemplary embodiment.

### [Mode for Invention]

Hereinafter, exemplary embodiments of the present disclosure will be described in detail with reference to the accompanying drawings so that those skilled in the art to which the present disclosure pertains may easily practice the present disclosure. The present disclosure may be implemented in various different forms and is not limited to exemplary embodiments provided herein.

Portions unrelated to the description will be omitted in order to obviously describe the present disclosure, and similar components will be denoted by the same reference numerals throughout the present specification. The term 'part' (portion) used in the specification may be implemented as software or hardware, and according to exemplary embodiments, multiple 'parts' may be implemented as one unit (element), or one 'part' may include multiple elements. Hereinafter, operating principles and exemplary embodiments of the present disclosure are described with reference to the attached drawings below.

In addition, the size and thickness of each component illustrated in the drawings are arbitrarily indicated for convenience of description, and the present disclosure is not necessarily limited to the illustrated those. In the drawings, the thickness of layers, films, panels, regions, etc., are exaggerated for clarity. In addition, in the accompanying drawings, thicknesses of some of layers and regions have been exaggerated for convenience of explanation.

In addition, it will be understood that when an element such as a layer, film, region, or substrate is referred to as being "on" another element, it can be directly on the other element or intervening elements may also be present. In contrast, when an element is referred to as being "directly on" another element, there are no intervening elements present. In addition, when an element is referred to as being "on" a reference element, it can be positioned on or beneath the reference element, and is not necessarily positioned on the reference element in an opposite direction to gravity.

In addition, unless explicitly described to the contrary, the word "comprise", and variations such as "comprises" or "comprising", will be understood to imply the inclusion of stated elements but not the exclusion of any other elements.

Further, throughout the specification, the word "plane" refers to a view when a target is viewed from the top, and the word "cross section" refers to a view when a cross section of a target taken along a vertical direction is viewed from the side.

In addition, terms including an ordinal number such as first, second, or the like, used in the present disclosure may be used to describe various components. However, these components are not limited to these terms. The above terms are used solely for the purpose of distinguishing one component from another.

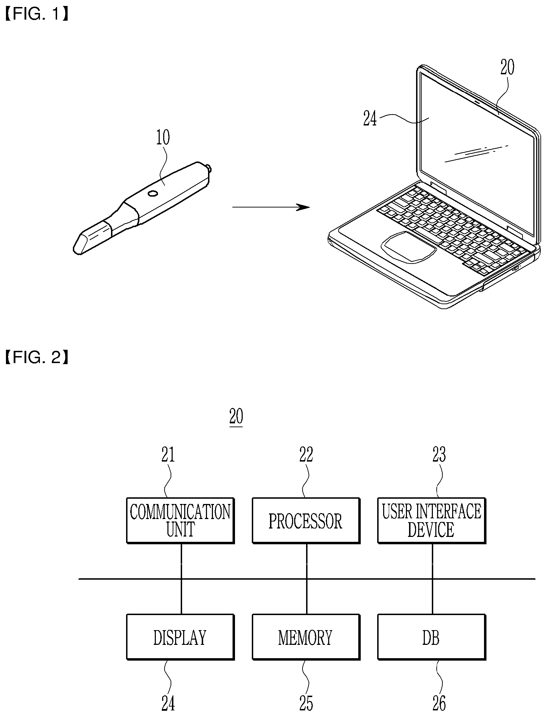

FIG. 1 is a diagram for describing a system for processing an image including an electronic apparatus according to an exemplary embodiment. FIG. 2 is a block diagram illustrating a configuration of an electronic apparatus according to an exemplary embodiment.

Referring to FIG. 1 and FIG. 2, a system 1 for processing an image may include a scanner 10 and an electronic apparatus 20.

In this specification, an 'object' is a capturing subject and may include a person, an animal, or a part thereof. For example, the object may include a part (an organ, an organ, etc.) of a body, a phantom, etc. In addition, for example, the object may include a plaster model modeling an oral cavity, a denture such as a denture or a prosthesis, a dentiform in a shape of teeth, etc. For example, the object may include a tooth, a gingiva, at least a portion of the oral cavity, and/or artificial structures (e.g., an orthodontic device including bracket and wire, dental restorations including implant, abutment, artificial teeth, inlay and onlay, and orthodontic auxiliary tools inserted into the oral cavity, etc.) that may be inserted into the oral cavity, the tooth or gingiva to which the artificial structures are attached, etc.

The scanner 10 may mean a device that acquires an image related to the object. The scanner 10 may mean a scanner 10 that acquires an intraoral image related to the oral cavity used for oral treatment. The scanner 10 may acquire at least one of a two-dimensional (2D) image and a three-dimensional (3D) image. In addition, the scanner 10 may acquire at least one 2D image of the oral cavity, and generate a 3D image (or a 3D model) of the oral cavity based on at least one acquired 2D image. In addition, the scanner may acquire at least one 2D of the oral cavity, and transmit the at least one 2D to the electronic apparatus 20.

The electronic apparatus 20 may also image a surface of at least one of the scanner 10 tooth model or tooth, a gingiva, and the artificial structures (e.g., the orthodontic device including the bracket and wire, the orthodontic auxiliary tools inserted into the oral cavity including the implant, the artificial teeth, and splint, etc.) insertable into the oral cavity, and for this purpose, may acquire surface information about the object as raw data.

The electronic apparatus 20 may generate the 3D image of the oral cavity based on at least one received 2D image. Here, the '3D image' may be generated by three-dimensionally modeling the object based on the received raw data, and thus may be called a '3D model'. In addition, in the present disclosure, a model or image representing an object two-dimensionally or three-dimensionally may be collectively called an 'image'.

For example, the scanner 10 may be an intraoral scanner having a form that may be inserted into the oral cavity, and according to the exemplary embodiment, the intraoral scanner may be a wired device or a wireless device, and the technical idea of the present disclosure is not limited to the form of the intraoral scanner.

According to an exemplary embodiment, the intraoral scanner may be a hand-held type scanner that can be held by hand and carried. The intraoral scanner may be inserted into the oral cavity, and scan teeth in a non-contact manner to obtain an image of the oral cavity including at least one tooth, and scan the inside of the patient's oral cavity using at least one image sensor (e.g., an optical camera, etc.).

According to an exemplary embodiment, the scanner 10 may be a table type scanner that may be used for dental treatment. The table type scanner may be a scanner that acquires the surface information on an object as the raw data by scanning the object using the rotation of the table. The table scanner may scan a surface of an object such as a plaster model or an impression model modeling the oral cavity.

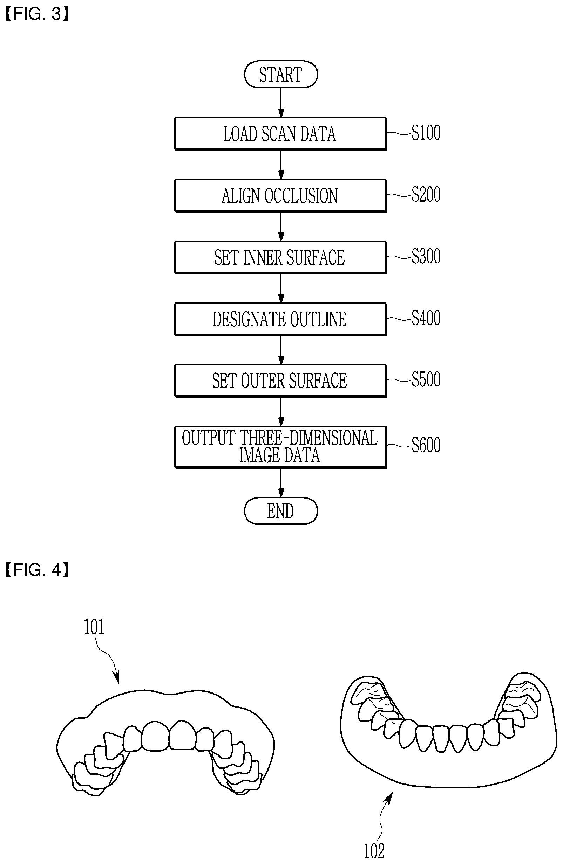

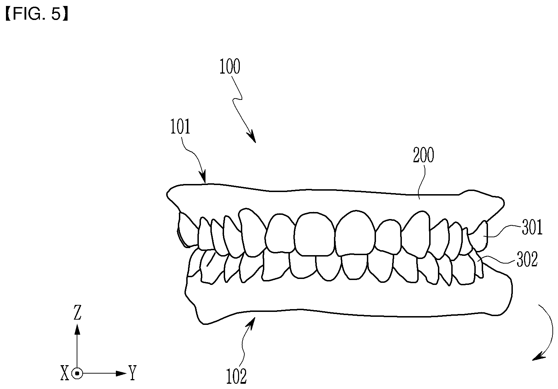

The electronic apparatus 20 may receive the raw data from the scanner 10 and process the received raw data to output a 3D image for the raw data. According to an exemplary embodiment, the output 3D image may be 3D image data for the splint for the received raw data. For ease of description, a specific description of the scan data is described later with reference to FIGS. 4 and 5.

The electronic apparatus 20 may be any electronic apparatus that is connected to the scanner via a wired or wireless communication network, and may receive a 2D image acquired by scanning the object from the scanner and generate, process, display, and/or transmit an image based on the received 2D image.

The electronic apparatus 20 may store and execute dedicated software to perform at least one operation of receiving, processing, storing, and/or transmitting the 3D image or the 2D image of the object. For example, the dedicated software may perform processing operations such as region extraction and region setting on the received scan data, and perform data selection, reference point adjustment, alignment, etc., based on the processing operations to perform at least one operation such as generation, storing, and transmitting the 3D image including the artificial structures such as the splint. The electronic apparatus 20 may be a computing device such as a smart phone, a laptop computer, a desktop computer, a PDA, or a tablet PC, but is not limited thereto. In addition, the electronic apparatus 20 may exist in the form of a server (or server device) for processing intraoral images.

The electronic apparatus 20 may include a communication unit 21, a processor 22, a user interface device 23, a display 24, a memory 25, and a database 26. However, not all of the illustrated components are essential components. The electronic apparatus 20 may be implemented by more components than the illustrated components, or may be implemented by fewer components. The components will be described below.

The communication unit 21 may perform communication with an external device. Specifically, the communication unit 21 may be connected to a network by wire or wirelessly to perform communication with the external device. Here, the external device may be the scanner 10, a server, a smartphone, a tablet, a PC, etc.

The communication unit 21 may include a communication module that supports one of various wired and wireless communication methods. For example, the communication module may be in the form of a chipset, or may be a sticker/barcode (e.g., a sticker including an NFC tag), etc., including information necessary for communication. In addition, the communication module may be a short-range communication module or a wired communication module.

For example, the communication unit 21 may support at least one of wireless LAN, wireless fidelity, Wi-Fi direct, Bluetooth, Bluetooth low energy, wired LAN, near field communication, Zigbee, Infrared data association (IrDA), 3G, 4G, and 5G.

In an exemplary embodiment, the scanner 10 may transmit the acquired raw data to the electronic apparatus 20 through the communication module. The image data acquired by the scanner may be transmitted to the electronic apparatus 20 connected through the wired or wireless communication network.

The processor 22 controls the overall operation of the electronic apparatus 20 and may include at least one processor, such as a CPU. The processor 22 may include at least one specialized processor corresponding to each function, or may be a processor integrated into one.

The processor 22 may receive the raw data through the communication unit 21. For example, the processor 22 may receive the raw data from the scanner 10 through the communication unit 21. In this case, the processor 22 may generate the 3D image data (e.g., surface data, mesh data, etc.) that represents the shape of the surface of the object three-dimensionally based on the received raw data. Hereinafter, the scan data that becomes the calculation target of the electronic apparatus 20 may include the 3D image data.

The processor 22 may receive library data from the external device through the communication unit 21. The library data may be data pre-stored in the electronic apparatus 20 or the raw data or the 3D image data acquired through the external device, but is not limited thereto. Here, the external device may be a camera capable of capturing pictures or videos, or an electronic apparatus having a camera function. In addition, the external device may be an intraoral scanner capable of scanning the inside of a patient's mouth.

The processor 22 may control the user interface device 23 or the display 24 to receive a predetermined command or data from a user.

The processor 22 may execute a program stored in the memory 25, read an image, data, or file stored in the memory 25, or store a new file in the memory 25. The processor 22 may execute instructions stored in the memory 25. The stored program may include, but is not limited to, dedicated software.

The processor 22 may set an inner surface of the splint for the intraoral image. According to an exemplary embodiment, the processor 22 may set the inner surface of the splint based on an input inner surface offset distance, surface smoothness, etc. The inner surface offset distance may mean a normal distance between the intraoral image and the inner surface of the splint. The surface smoothness may mean the roughness of the inner surface of the splint.

In addition, according to an exemplary embodiment, an inner surface of the intraoral image may be established as a result of an object recognition inference operation of the processor 22.

The processor 22 may designate a splint outline for the intraoral image. According to an exemplary embodiment, the processor 22 may designate the splint outline based on an input buccal height, a lingual height, etc. The buccal height may mean a height formed along an outer wall of tooth based on a bottom surface of a tooth region, and the lingual height may mean a height formed along an inner wall of tooth based on the bottom surface of the tooth region of the intraoral image.

In addition, according to an exemplary embodiment, the splint outline for the intraoral image may be designated as a result of the object recognition inference operation of the processor 22. According to an exemplary embodiment, the splint outline may be designated manually using the user interface device 23.

The processor 22 may generate a crop image based on the set inner surface and the designated outline. The crop image is the 3D image data, and may be generated by cutting out an outer portion of the splint outline for the inner surface set based on the intraoral image.

The processor 22 may perform a calculation operation on mesh data, data, etc., included in the image data. For example, the processor 22 may perform Laplacian smoothing on image data, perform a Convex hull algorithm, or generate image intersections using a ray tracing algorithm to generate a distance to antagonist between objects and a vector corresponding to the distance to antagonist, and/or perform radial basis function (RBF) interpolation based on a plurality of points.

The Laplacian smoothing is an algorithm for smoothing a polygonal mesh, and may adjust and move a new location of each vertex of the mesh based on local information (e.g., adjacent locations), and may utilize an umbrella operator and a shrink-wrap algorithm.

The Convex hull algorithm is an algorithm for generating a convex polyhedron containing points in a finite set of points, and according to an exemplary embodiment, a static calculation algorithm or a dynamic calculation algorithm utilizing triangulation may be used.

The image intersection may be a point where a straight line such as a ray is transmitted through the image data using the ray tracing algorithm, but is not limited thereto. The distance to antagonist between the objects may be found by comparing lengths generated between the image intersections of the objects, and the vector direction corresponding to the distance to antagonist may be calculated.

The processor 22 may recognize an object in the scan data, extract a portion of a region, or calculate the area or volume of the recognized object or the extracted region. For example, the processor 22 may separately recognize the type of teeth by using a curvature information, cusp information, etc., of the scan data, or distinguish a space between teeth. The cusp information may include the number and arrangement of cusp points where a molar in the scan data comes into contact. According to an exemplary embodiment, the recognition operations of the processor 22 are not limited to the examples of utilizing the information, and the recognition operations may be performed through the inference of the object recognition artificial intelligence algorithm. For example, in the scan data for the intraoral image, the processor 22 may recognize the type and number of teeth in the scan data through the object recognition artificial intelligence algorithm.

The processor 22 may determine and adjust a spacing between the image data and the corresponding image data of the inner surface of the splint as a splint thickness, and set an outer surface of the splint by adjusting the image data.

The processor 22 may select data using predetermined values, etc., as reference and adjust the image data. For example, the processor 22 may calculate the thickness through the distance between the image data, and when the calculated thickness is greater than the predetermined value, perform an operation of adjusting the position of the image data to adjust the thickness.

The processor 22 may generate the 3D image data for the splint using the inner surface and the outline of the splint and the outer surface of the splint set based on the inner surface and outline.

The user interface device 23 may mean a device that receives data from a user to control the electronic apparatus 20. The display 24 may include an output device for displaying a result image according to the operation of the electronic apparatus 20 or the 3D image output from the electronic apparatus 20.

The user interface device 23 may include, for example, an input device such as a mouse, a joystick, an operation panel, a touch sensitive panel that receives user input, and the display 24 may include a display panel that displays a screen, etc.

The database 26 may store data and a dataset for training an artificial intelligence algorithm of dedicated software, and may provide data for training according to a request of the dedicated software. The artificial intelligence algorithm may train the training data of teeth stored in the database 26 using a deep learning method and distinguish the characteristics of data representing teeth. Meanwhile, the dedicated software may use the extracted or recognized tooth region data when performing an occlusal plane alignment step, an inner setting step, an outline designation step, etc., which will be described later, by extracting maxillary tooth region data and mandibular tooth region data from scan data or recognizing objects according to tooth characteristics.

In the present disclosure, the artificial intelligence (AI) means a technology that imitates human learning ability, reasoning ability, and perception ability and implements them with a computer, and may include the concepts of machine learning and symbolic logic. The machine learning (ML) may be an algorithm technology that classifies or trains the characteristics of input data on its own. The technology of the artificial intelligence may analyze input data as the machine learning algorithm, train the results of the analysis, and make the judgment or prediction based on the results of the training. In addition, technologies that imitate the cognitive and judgment functions of the human brain by utilizing the machine learning algorithm may also be understood as part of the category of the artificial intelligence. For example, the fields of technology of linguistic understanding, visual understanding, inference/prediction, knowledge expression, and motion control may be included.

In this disclosure, the machine learning may mean a process of training a neural network model using experience in processing data. Through the machine learning, the computer software may mean improving its own data processing ability. A neural network model is constructed by modeling correlations between data, and the correlations may be expressed by multiple parameters. The neural network model extracts and analyzes features from given data to derive correlations between data, and repeats the process to optimize the parameters of the neural network model, which may be called the machine learning.

For example, the neural network model may train a mapping (correlation) between inputs and outputs for data given as input-output pairs. Alternatively, even when only the input data is given, the neural network model may derive regularities between the given data and train the relationship.

In the present disclosure, the artificial intelligence training model, the machine learning model, or the neural network model may be designed to implement a human brain structure on a computer, and may include a plurality of network nodes that simulate neurons of a human neural network and have weights. The plurality of network nodes may simulate synaptic activity of neurons that exchange signals through synapses, and thus may have a connection relationship between each other. In the artificial intelligence learning model, the plurality of network nodes may be located in layers of different depths and may exchange data according to the convolution connection relationship.

Although the database 26 is illustrated as being included in the electronic apparatus 20 in the drawing, it is not limited thereto and may be arranged in the form of a server (or server device) or the like outside the electronic apparatus 20 to provide data for training and store training results.

FIG. 3 is a flowchart illustrating a method for processing an image of an electronic apparatus according to an exemplary embodiment. FIGS. 4 and 5 are diagrams for describing scan data, maxillary scan data, and mandibular scan data according to an exemplary embodiment.

Referring to FIGS. 1 to 5, the electronic apparatus 20 loads scan data 100 (S100). The electronic apparatus 20 loads the scan data 100 generated based on the image received from the external device including the scanner 10, etc., through the communication unit 21.

The electronic apparatus 20 loads scan data 101 processed based on the received image or stored in the processor 22 or the user interface device 23, and may display the loaded scan data 101 through the display 24.

The scan data 100 may be the 2D image of the object, the 3D model representing the object in three dimensions, or the 3D image data, and specifically, may be a 3D intraoral model.

According to an exemplary embodiment, the intraoral images in FIGS. 4 and 5 correspond to the scan data 100 and are 2D or 3D expressions of the objects of the scan data 100, and may include a maxillary pre-preparation (prep) image, a maxillary prep image, a mandibular pre-prep image, a mandibular prep image, an occlusal image including a maxillary-related image and a mandibular-related image, as in the scan data 100 described below.

In the present disclosure, the prep may mean a series of preparatory processes for removing a portion of the enamel and dentin of the teeth so as to prevent interference between natural teeth and splints when performing prosthetics such as crowns and bridges.

A "3D intraoral model" may mean a model that three-dimensionally models the oral cavity based on the raw data acquired by the scanning operation of the scanner. In addition, the "3D intraoral model" may mean a structure that is three-dimensionally modeled based on the data acquired by scanning an object such as a tooth, an impression, and an artifact. The 3D intraoral model is generated by modeling the internal structure of the oral cavity in three dimensions, and may be called a 3D scan model, a 3D model, or a tooth model. For example, a format of the 3D intraoral model may be one of standard triangle language (STL), OBJ, and polygon file formats, and is not limited to the above examples. In addition, the 3D intraoral model may include information such as geometric information, color, texture, and a material for a 3D shape.

In addition, the "polygon" may mean a polygon which is the smallest unit used when expressing the 3D shape of the 3D intraoral model. For example, the surface of the 3D intraoral model may be expressed as triangular polygons. For example, a polygon may be composed of at least three vertices and one face. A vertex may include information such as location, color, and normal. A mesh may be an object in a 3D space created by gathering multiple polygons. As the number of polygons representing the 3D intraoral model increases, the object may be expressed in detail.

The scan data 100 may include at least one of the maxillary scan data 101 and the mandibular scan data 102. Specifically, the scan data 100 may load any one of the maxillary pre-prep data, the maxillary prep data, the mandibular pre-prep data, the mandibular prep data, and the occlusal data including the maxillary-related data and the mandibular-related data.

In the present disclosure, the prep means a series of preparatory processes for removing a portion of enamel and dentin of the tooth so as to prevent interference between the natural tooth and the prosthesis when performing prosthetics such as crowns and bridges. The prep data may be data in which the enamel and dentin of the tooth are removed through the preparatory process, and the pre-prep data may be data before a portion of enamel and dentin of the tooth are removed through the preparatory process.

The scan data 100 may include a gingival region 200, maxillary tooth region data 301, and mandibular tooth region data 302, which are arranged in the maxillary scan data 101 and the mandibular scan data 102.

The electronic apparatus 20 may load at least one of the maxillary scan data 101, the mandibular scan data 102, and the occlusal data including the maxillary scan data 101 and the mandibular scan data 102.

The electronic apparatus 20 analyzes and aligns the shape of the received scan data 100 (S200). In the corresponding step, an occlusal plane and a midline for the scan data 100 are set, and the electronic apparatus 20 may automatically align the scan data 100, the maxillary scan data 101, or the mandibular scan data 102 according to the occlusal plane, and may display the left and right alignment by a midline through the display 24.

In addition, at the corresponding stage, the user may manually designate a reference point on the scan data 100 to set the front direction and the occlusal plane of the scan data 100, and align the scan data 100 along the set occlusal plane. For example, the user may select some data of the scan data 100 through the user interface device 23 at the corresponding step, and align the scan data 100 with the selected data as a reference point.

The electronic apparatus 20 sets the inner surface of the splint for the aligned scan data 100 (S300).

At the corresponding step, the electronic apparatus 20 may designate the direction in which the splint is to be inserted by considering the undercut of the aligned scan data 100. For example, when manufacturing the splint, the electronic apparatus 20 may calculate the area of the tooth region in the scan data 100, and designate the direction in which the splint is to be inserted by considering the undercut and block out according to the direction in which the splint is to be inserted. The insertion efficiency and retention force of the splint may be improved by designating the insertion direction of the splint as described above.

Based on the inner surface offset distance, the surface smoothness, etc., input from the user interface device 23, the electronic apparatus 20 may set the inner surface of the splint to be output. The inner surface offset distance may mean a separation distance in the normal direction between the scan data 100 and the inner surface of the splint. The surface smoothness may mean the roughness of the inner surface of the splint.

The electronic apparatus 20 designates the outline of the splint for the automatically aligned scan data 100 (S400).

Based on the buccal height, the lingual height, etc., input from the user interface device 23, the electronic apparatus 20 may designate the outline of the splint to be output. For example, when manufacturing the splint, the buccal height is a height of the outer wall of the tooth facing a cheek based on a lower surface of the tooth region, and for example, the buccal height may mean a height formed along the outer wall of the tooth based on the bottom surface of the tooth region of the maxillary scan data 101. The higher the buccal height, the closer the buccal outline formed is to the gingiva. The lingual height is a height of the inner wall of the tooth facing a tongue based on a bottom surface of the tooth region, and for example, the lingual height may mean a height formed along the inner wall of the tooth based on the bottom surface of the tooth region of the maxillary scan data 101. The higher the lingual height, the closer the lingual outline formed is to the gingiva.

The electronic apparatus 20 sets the outer surface of the splint for the aligned scan data 100 (S500).

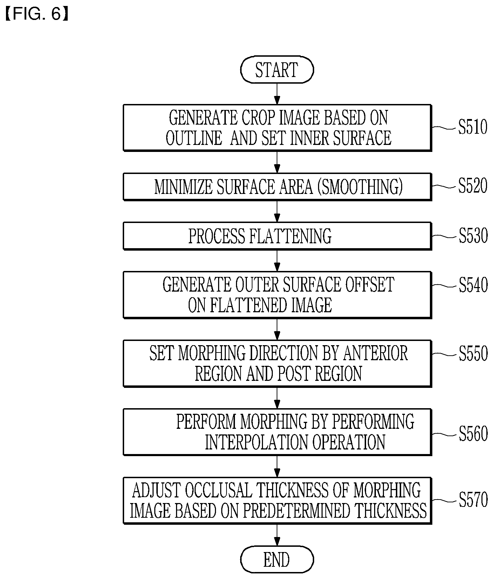

Based on the thickness, the surface smoothness, etc., input from the user interface device 23, the electronic apparatus 20 may designate the outer surface of the splint to be output. The electronic apparatus 20 may form the 3D image data for the splint by setting the thickness of the splint in the occlusal direction based on the predetermined occlusal thickness. For example, when manufacturing the splint, the thickness may mean the thickness from the inner surface of the splint in the buccal/lingual direction. The surface smoothness may mean the roughness of the outer surface of the splint. The predetermined occlusal thickness may mean the maximum thickness value that the splint extends in the occlusal direction. A specific description of the corresponding step will be described later in the description of FIGS. 6 to 18.

The electronic apparatus 20 generates the 3D image data through the information set and designated in steps S300 to S500 (S600). The 3D image of the generated splint may be transmitted to the external device through the communication unit 21 and output to the splint. The external device may be a 3D printer, but is not limited to the above example according to an exemplary embodiment.

According to an exemplary embodiment, the electronic apparatus 20 may perform steps S200 to S500 at once without an intermediate input from the user. The electronic apparatus 20 loads the scan data 100 (S100), receives inputs, such as the inner surface offset distance, the surface smoothness, the buccal height, the lingual height, and the thickness, from the user, and automatically performs steps S200 to S500 without intermediate intervention from the user to generate the 3D image data (S600).

According to an exemplary embodiment, the electronic apparatus 20 may automatically perform steps S200 to S500 without user intervention by utilizing the inner surface offset distance, the surface smoothness, the buccal height, the lingual height, the thickness, etc., stored in the memory 25.

The electronic apparatus 20 may automatically perform steps S200 to S500 without the user intervention, thereby reducing the time required for the splint manufacturing.

According to an exemplary embodiment, the electronic apparatus 20 trains the neural network model within the artificial intelligence algorithm while processing the plurality of scan data, and the electronic apparatus 20 may generate the 3D image data by inferring about scan data 100 loaded without the user intervention through the artificial intelligence algorithm (S600).

According to an exemplary embodiment, the electronic apparatus 20 may adjust an occlusal state or a distance to antagonist between arches in each step of steps S300 to S500 through the user input regarding the occlusal state or the distance to antagonist between the arches between the maxillary scan data 101 and the mandibular scan data 102 in the scan data 100. During the adjustment operation, the electronic apparatus 20 may perform the calculation operation on the scan data 100 to display the occlusal state or the distance to antagonist between the arches together.

Through the adjustment and calculation operations, the user may produce the splint by considering the distance and space in the patient's oral cavity.

Hereinafter, step S500 will be described with reference to FIGS. 6 to 18.

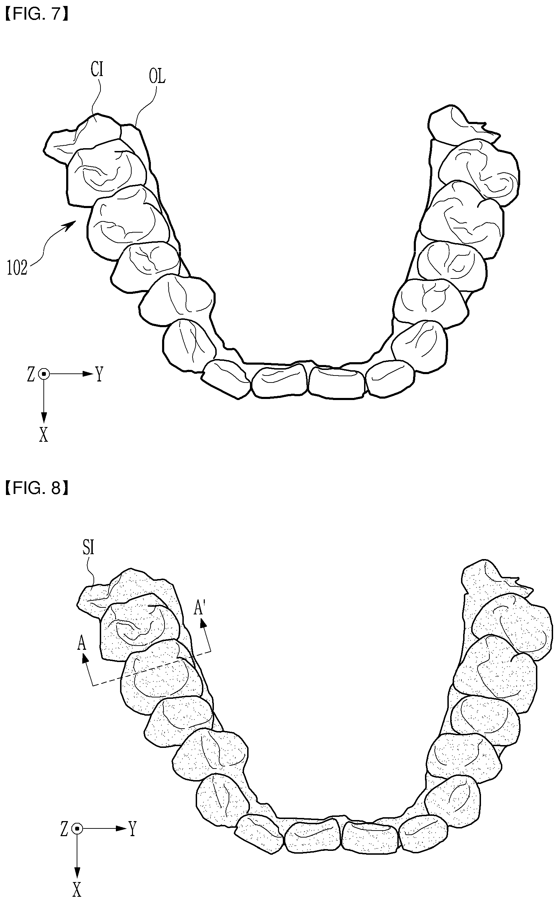

FIG. 6 is a flowchart showing a method for processing an image of an electronic apparatus according to an exemplary embodiment. FIG. 7 is a diagram for describing a step of generating a crop image according to an exemplary embodiment.

Referring to FIGS. 1 to 7, the electronic apparatus 20 may set a crop image Cl based on the inner surface of the splint set in step S300 for the scan data 100 and a splint outline OL designated in step S400 for the scan data 100 (S510).

The crop image Cl is the 3D image data and may be generated by cutting out the outer portion of the splint outline OL for the inner surface set based on the scan data 100. The scan data 100 and the crop image Cl may be separated by an inner surface offset distance set in the normal direction of the scan data 100.

In general, the intraoral image scanned through the 3D scanner often includes regions that need to be removed, where objects other than the object to be measured are scanned together and have irrelevant or inaccurate information. Such scan regions are referred to as artificial data or flap regions that may represent a portion of the patient's lips, cheeks, tongue, or retromolar area. By generating the crop image Cl, unnecessary data for the splint manufacturing may be removed in advance, thereby increasing the efficiency of the splint manufacturing.

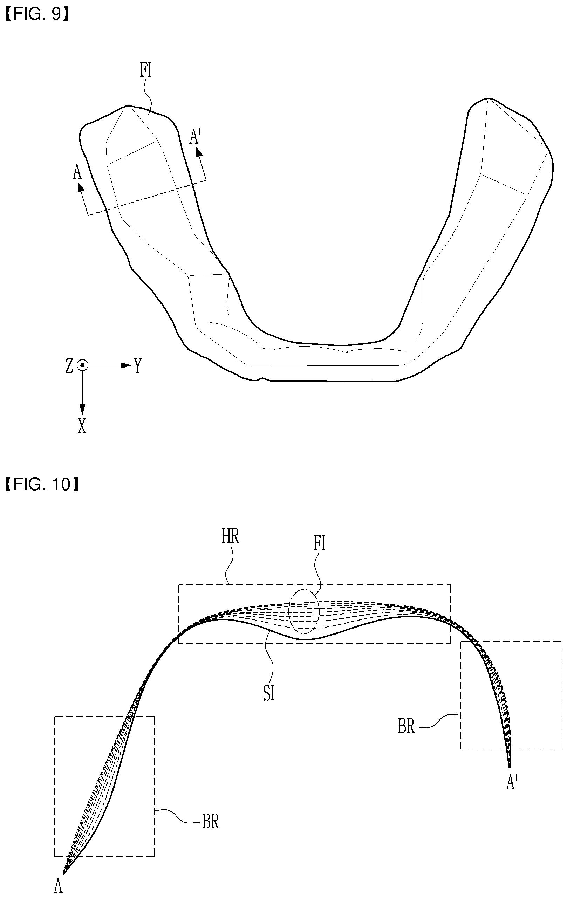

In FIG. 7, the generation of the crop image Cl is described using the mandibular scan data 102 of the scan data 100 as an example, but may vary according to the exemplary embodiment and is not limited to the above example. Hereinafter, the description of the mandibular scan data 102 in FIGS. 9 to 18 may also be applied to other scan data 100 including the maxillary scan data 101, and for ease of description, the description of the other scan data 100 will be described focusing on differences from the description of the mandibular scan data 102.

FIG. 8 is a diagram for describing a step of generating a smoothing image according to an exemplary embodiment.

Referring additionally to FIG. 8, the electronic apparatus 20 performs a smoothing operation that minimizes the surface area of the crop image CI, thereby generating a smoothing image SI (S520).

The electronic apparatus 20 may perform the Laplacian smoothing that minimizes the surface area by minimizing the curvature of the surfaces for the crop image Cl. The Laplacian smoothing may adjust and move a new position of a vertex based on local information (e.g., adjacent positions) for each vertex, and may utilize, but is not limited to, an umbrella operator and a shrink-wrap algorithm. In addition, the Laplacian smoothing may be used as an example to perform the smoothing operation on the crop image Cl, but the technical idea of the present disclosure is not limited thereto.

The electronic apparatus 20 may generate the smoothing image SI through the Laplacian smoothing.

FIGS. 9 and 10 are diagrams for describing a step of generating a flattened image according to an exemplary embodiment. FIG. 10 is a cross-sectional view of the smoothing image and the flattened image cut along A-A' of FIGS. 8 and 9, and is a diagram for describing the smoothing image and the flattened image.

Referring additionally to FIGS. 9 and 10, the electronic apparatus 20 flattens the smoothing image SI to generate the flattened image FI (S530).

According to an exemplary embodiment, the electronic apparatus 20 may generate a convex hull for a vertex in the smoothing image SI through the convex hull algorithm, and correct the vertex in the smoothing image SI according to the characteristics of the convex hull according to the generated convex hull. The corrected vertex may be included in the convex hull.

In the present disclosure, the characteristics of the convex hull may mean moving the position by giving a weight to the distance from the vertex to the outermost surface of the convex hull generated by the convex hull algorithm.

The electronic apparatus 20 may generate a Bezier curve by differentiating the weight according to the region for the vertex in the smoothing image SI, and correct the Bezier curve, and generate the flattened image FI.

The electronic apparatus 20 may perform correction by changing weights on a boundary region BR of the smoothing image SI and a higher region HR of the smoothing image SI, and generate the flattened image FI. The electronic apparatus 20 may apply a boundary flat weight to the boundary region BR and may apply a flat weight to the higher region HR. The boundary flat weight may be 0.0 to 3.0, specifically 0.0 to 1.0. The flat weight may be 0.0 to 3.0, specifically 0.0 to 1.0. According to an exemplary embodiment, the values of the boundary flat weight and the flat weight may be the same or different.

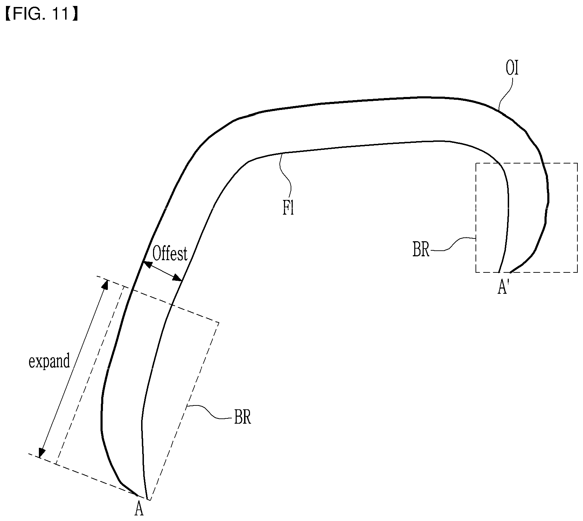

FIG. 11 is a diagram for describing a step of generating the offset image for generating the outer surface of the splint according to an exemplary embodiment. FIG. 11 illustrates an offset image based on a cross-sectional view cut along line A-A' of the flattened image of FIG. 9.

Referring additionally to FIG. 11, the electronic apparatus 20 offsets the flattened image FI to generate an outer surface offset image OI (S540).

The electronic apparatus 20 may move the vertex in the flattened image FI by a constant value in the normal direction and may move the vertex in the boundary region BR by a thinner interval than other regions to generate the offset image OI. The electronic apparatus 20 may move the vertex in the boundary region BR of the flattened image FI by making the outer offset thinner as it approaches the outline. Therefore, a distance between the offset image OI and the flattened image FI may become thinner as it approaches the outline.

According to an exemplary embodiment, the electronic apparatus 20 may set a plurality of reference points based on a section length (expand) and an outer surface offset of the boundary region BR and generate a Bezier curve based on the plurality of reference points to generate the offset image OI of the boundary region BR. However, the boundary region BR of the offset image OI may be generated by a method other than the above method.

According to an exemplary embodiment, the electronic apparatus 20 may receive the information on the outer surface offset in advance through the user interface device 23, and the outer surface offset may correspond to the splint thickness in the buccal/lingual direction as a gap between the flattened image FI and the offset image OI.

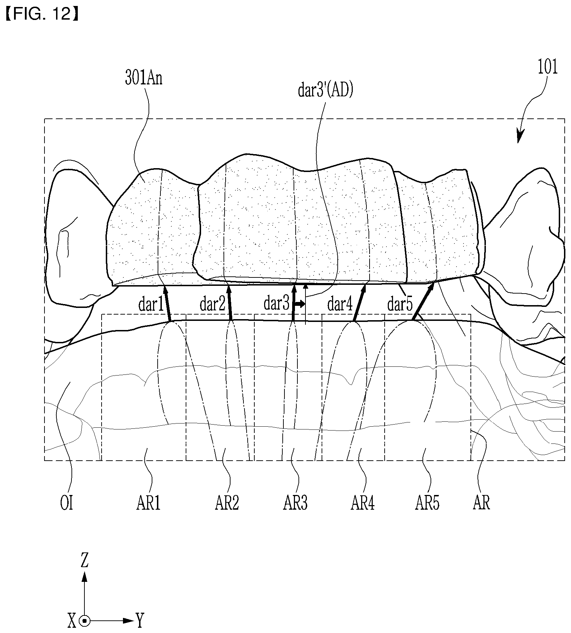

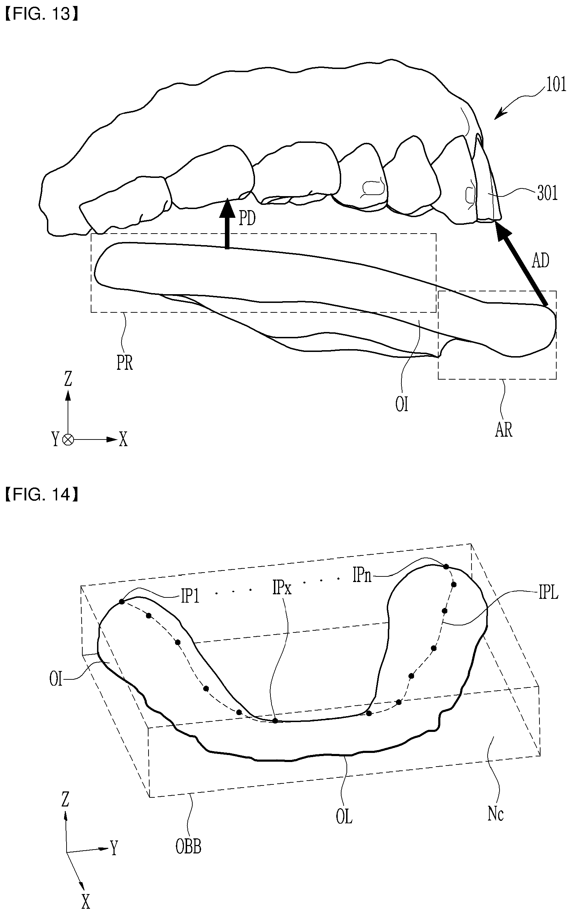

FIGS. 12 and 13 are diagrams for describing a step of setting a morphing direction according to an exemplary embodiment.

Referring additionally to FIGS. 12 and 13, the electronic apparatus 20 sets a morphing direction by distinguishing an anterior region AR and a post region PR (S550).

The electronic apparatus 20 may assume a distance to antagonist between the arches between the maxillary scan data 101 and the mandibular scan data 102 and adjust the position of the maxillary scan data 101 and the offset image OI. The assumed distance to antagonist between the arches between the maxillary scan data 101 and the mandibular scan data 102 may be greater than the outer surface offset value of the offset image OI.

As illustrated in FIG. 13, the electronic apparatus 20 may fix the maxillary scan data 101 on the occlusal plane and adjust the position of the offset image OI, but may fix the mandibular scan data 102 on the occlusal plane and adjust the position of the offset image OI.

The offset image OI may include the anterior region AR and the post region PR. The anterior region AR and the post region PR of the offset image OI may be images generated based on the shapes of the anterior and post of the oral cavity. Similarly, the anterior region AR and the post region PR of the offset image OI and the anterior and post regions of the crop image CI may correspond to each other, the anterior region AR and the post region PR of the offset image OI and the anterior and post regions of the smoothing image SI may also correspond to each other, and the anterior region AR and the post region PR of the offset image OI and the anterior and post regions of the flattened image FI may also correspond to each other.

The anterior region AR may include a plurality of partial anterior regions AR1 to AR5 that are divided from each other and do not overlap each other. The electronic apparatus 20 may generate each distance to antagonist vector dar1 to dar5 having a distance to antagonist from the maxillary anterior region 301An of the maxillary scan data 101 for each of the plurality of partial anterior regions AR1 to AR5. For example, a size of a third distance to antagonist vector dar3 of the third partial anterior region AR3 among the distance to antagonist vectors dar1 to dar5 of the plurality of partial anterior regions AR1 to AR5 may be the smallest.

The electronic apparatus 20 may project the third distance to antagonist vector dar3 onto an XZ plane generated based on a first direction X parallel to the front direction of the scan data 100 and a third direction Z parallel to the normal vector of the occlusal plane, based on the front direction of the scan data 100 and the normal vector of the occlusal plane set in step S200. The electronic apparatus 20 may set the direction of the third distance to antagonist vector dar3' projected onto the XZ plane to a morphing direction AD of the anterior region.

In FIG. 12, the plurality of partial anterior regions AR1 to AR5 are described as five, but the technical idea of the present disclosure is not limited to the number of the plurality of partial anterior regions, and the number may vary according to the exemplary embodiment.

The electronic apparatus 20 may set, as a post region morphing direction PD, the third direction Z which is parallel to the normal vector of the occlusal plane set in step S200, with respect to the post region PR.

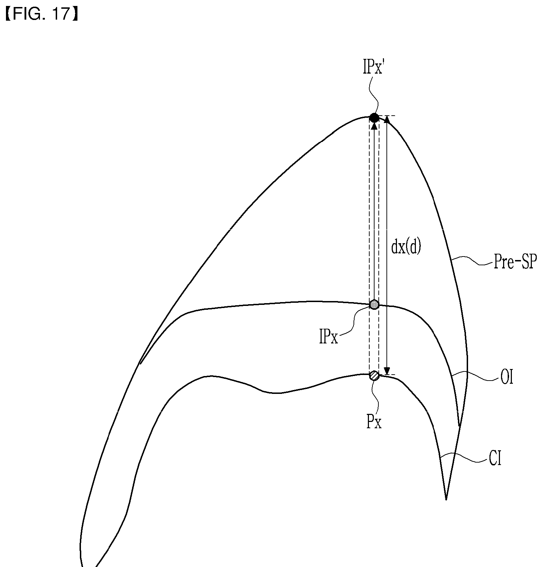

FIGS. 14 to 17 are diagrams for describing a morphing step according to one exemplary embodiment. FIG. 17 illustrates a cross-sectional view of the offset image and the morphing image cut in the morphing direction for describing the occlusal thickness.

Referring additionally to FIGS. 14 to 17, the electronic apparatus 20 performs the interpolation operation to perform the morphing (S560).

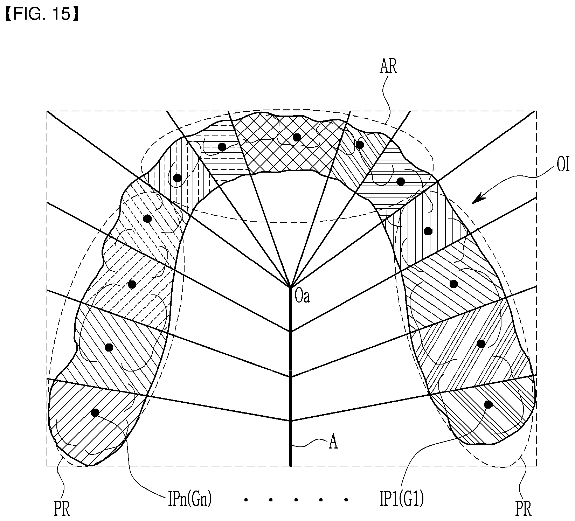

As illustrated in FIG. 15, the electronic apparatus 20 may divide the offset image OI into a plurality of image groups G1 to Gn. Among the plurality of image groups G1 to Gn, the image group corresponding to the anterior region AR may be divided with a center point Oa of an arch corresponding to the offset image OI as the axis. Among the plurality of image groups G1 to Gn, the image group corresponding to the post region PR may be divided with a center line A extending between the post regions PR of the offset image OI. The number of the plurality of image groups illustrated in FIG. 7 is an example for easy description, and the technical idea of the present disclosure is not limited to the number of the plurality of image groups.

As illustrated in FIG. 14, the electronic apparatus 20 may set a plurality of interpolation points IP1 to IPn for each of the plurality of image groups G1 to Gn. Each of the plurality of interpolation points IP1 to IPn may correspond to a vertex having a distance to antagonist from the maxillary scan data 101 in the plurality of image groups G1 to Gn. In the setting of the plurality of interpolation points IP1 to IPn, the electronic apparatus 20 may generate the image intersection of the maxillary scan data 101 generated through the ray tracing algorithm at a vertex in the plurality of image groups G1 to Gn, calculate a distance between the vertex and the image intersection, compare the distances between the plurality of vertices and the image intersections to obtain the distance to antagonist, and set the plurality of interpolation points IP1 to IPn.

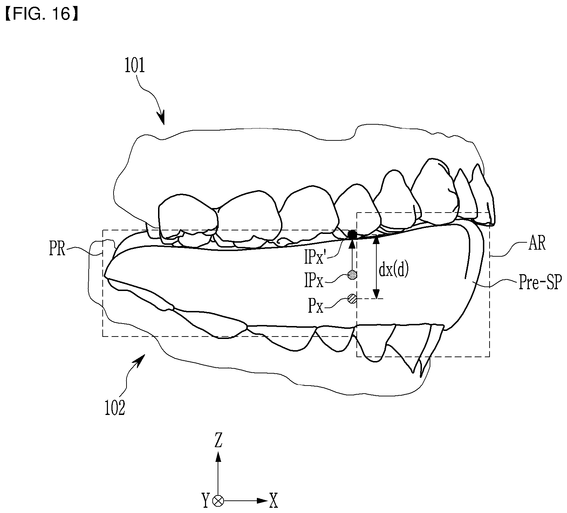

As illustrated in FIG. 16, the electronic apparatus 20 may perform the interpolation operation on the offset image OI based on the plurality of set interpolation points IP1 to IPn to generate a morphing image Pre-SP. The electronic apparatus 20 may perform the radial basis function (RBF) interpolation on the offset image OI based on a plurality of interpolation points IP1 to IPn.

The electronic apparatus 20 may move the plurality of interpolation points IP1 to IPn according to the morphing direction set in step S550. The interpolation point corresponding to anterior region AR among the plurality of interpolation points IP1 to IPn moves along the morphing direction AD of the anterior region, and the interpolation point corresponding to the post region PR among the plurality of interpolation points IP1 to IPn moves along the post region morphing direction PD. In addition to the movement of the plurality of interpolation points IP1 to IPn, the positions of vertices in the offset image OI may also change and be morphed during the interpolation operation.

The electronic apparatus 20 may move the plurality of interpolation points IP1 to IPn until they come into contact with the maxillary scan data 101. For example, among the plurality of interpolation points IP1 to IPn, an xth interpolation point IPx may move along the morphing direction to an xth contact point IPx' with the maxillary scan data 101.

According to an exemplary embodiment, an xth contact point IPx' in the morphing image Pre-SP corresponding to the post region PR of the offset image OI may correspond to the xth inner point Px of the crop image CI located in the negative direction -Z of the third direction Z, which is the normal direction of the occlusal plane which is the post region morphing direction PD, from the xth contact point IPx'. The electronic apparatus 20 may set an xth occlusal thickness dx, which is a distance between the xth contact point IPx' and the xth inner point Px, as the occlusal thickness.

According to an exemplary embodiment, one contact point corresponding to the anterior region AR of the offset image OI and arranged in the morphing image Pre-SP may correspond to one inner point arranged in the crop image CI in the morphing direction AD of the anterior region. The electronic apparatus 20 may set the distance between the above-described one contact point and the above-described one inner point as the occlusal thickness.

The method for processing an image of the present disclosure may reduce additional design processes while easily manufacturing the splint's contact with the molar through the morphing operation by performing the smoothing operation of step S520 and the flattening operation of step S530 before the morphing operation of step S560 to pre-process the surface shape of the tooth.

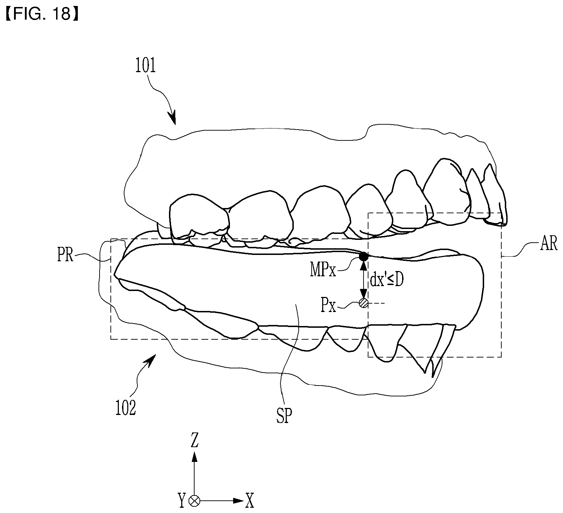

FIG. 18 is a diagram for describing a step of adjusting the occlusal thickness according to an exemplary embodiment.

Referring additionally to FIG. 18, the electronic apparatus 20 adjusts the occlusal thickness of the morphing image Pre-SP based on the predetermined thickness D (S570).

The electronic apparatus 20 may compare the occlusal thickness between the vertex in the morphing image Pre-SP and the vertex of the corresponding crop image CI based on the predetermined thickness D. For example, the x-th contact point IPx' of the morphing image Pre-SP corresponding to the post region PR of the offset image OI may correspond to the x-th inner point Px of the crop image CI in the third direction Z, which is the normal direction of the occlusal plane which is the post region morphing direction PD, and the x-th occlusal thickness dx, which is the distance between the x-th contact point IPx' and the x-th inner point Px, may be compared with the predetermined thickness D.

The electronic apparatus 20 may adjust the position of the vertex in the morphing image Pre-SP to adjust the occlusal thickness between the adjusted vertex of the morphing image Pre-SP and the vertex of the crop image CI to be less than or equal to the predetermined thickness D, when the occlusal thickness between the vertex in the morphing image Pre-SP and the corresponding vertex of the crop image CI is greater than the predetermined thickness D. The predetermined thickness D may be 3 mm to 10 mm, preferably 4 mm to 8 mm, and more preferably 6 mm to 8 mm.

According to an exemplary embodiment, the value of the predetermined thickness D may be input through the user interface device 23, and the technical idea of the present disclosure is not limited to the above numerical range.

For example, the electronic apparatus 20 may adjust the position of the x-contact point IPx' to the x-adjustment point MPx along the normal direction of the occlusal plane, which is the post region morphing direction PD, when the x-th occlusal thickness dx, which is the distance between the x-th contact point IPx' and the x-th inner point Px, is greater than the predetermined thickness D, thereby adjusting the occlusal thickness to the x-adjustment occlusal thickness dx' which is the distance between the x-th adjustment point MPx and the x-th inner point Px.

The electronic apparatus 20 may perform the adjustment operation for the occlusal thickness for the morphing image Pre-SP to generate the outer surface of the splint image SP.

The electronic apparatus 20 may generate the 3D image data for the splint generated based on the crop image CI, the outline OL, and the outer surface of the splint image SP.

The morphing operation of step S560 and the adjustment operation of step S570 are mainly described with respect to the post region PR and the post region morphing direction PD of the offset image OI, but the morphing operation of step S560 is also performed in the anterior region AR of the offset image OI in the anterior region morphing direction AD, and the morphing image Pre-SP corresponding to the anterior region AR may have the occlusal thickness formed in the anterior region morphing direction AD and may be adjusted in the anterior region morphing direction AD.

The electronic apparatus 20 may generate the outer surface of the splint image SP through the morphing operation and the adjustment operation.

The electronic apparatus 20 may display the intraoral image to which the splint is applied through the display 24, as illustrated in FIG. 18. In an exemplary embodiment, the user may select the maxilla or the mandible to which the splint is to be applied, and may display the intraoral image to which the splint is applied to the arch selected by the user.

The method for processing an image according to an exemplary embodiment of the present disclosure may be implemented in a form of program commands that may be executed through various computer means and may be recorded in a computer readable recording medium. In addition, an exemplary embodiment of the present disclosure may be a computer readable recording medium on which one or more programs including commands for executing an image processing method are recorded.

The computer readable medium may include a program command, a data file, a data structure, or the like, or a combination thereof. The program commands recorded in the computer readable recording medium may be especially designed and configured for the present disclosure or be known to those skilled in a field of computer software. Examples of the computer readable recording medium may include a magnetic medium such as a hard disk, a floppy disk, or a magnetic tape; an optical medium such as a compact disk read only memory (CD-ROM) or a digital versatile disk (DVD); a magneto-optical medium such as a floptical disk; and a hardware device specially configured to store and execute program commands, such as a ROM, a random access memory (RAM), a flash memory, or the like. Examples of the program commands include high-level language codes capable of being executed by a computer using an interpreter, or the like, as well as machine language codes made by a compiler.

Here, the machine-readable storage medium may be provided in a form of a non-transitory storage medium. Here, the "non-transitory storage medium" means that the storage medium is a tangible device, and does not include a signal (for example, electromagnetic waves), and the term does not distinguish between the case where data is stored semi-permanently on a storage medium and the case where data is temporarily stored thereon. For example, the "non-transitory storage medium" may include a buffer in which data is temporarily stored.

According to an embodiment, the methods according to various embodiments disclosed in the document may be included in a computer program product and provided. The computer program product may be traded as a product between a seller and a purchaser. The computer program product may be distributed in the form of a machine-readable storage medium (for example, compact disc read only memory (CD-ROM)), or may be distributed through an application store (for example, Play Store^{™}) or may be directly distributed (for example, download or upload) between two user devices (for example, smart phones) online. In a case of the online distribution, at least some of the computer program products (for example, downloadable app) may be at least temporarily stored in a machine-readable storage medium such as a memory of a server of a manufacturer, a server of an application store, or a relay server or be temporarily created.

Although exemplary embodiments of the present disclosure have been described in detail hereinabove, the scope of the present disclosure is not limited thereto, but may include several modifications and alterations made by those skilled in the art using a basic concept of the present disclosure as defined in the claims.

## Claims

1. A method for processing an image, comprising:
receiving at least one intraoral image;
aligning the at least one intraoral image to an occlusal plane;
setting an inner surface of a splint based on the at least one aligned intraoral image;
setting an outline of the splint based on the at least one intraoral image;
setting an outer surface of the splint based on the outline; and
displaying the at least one intraoral image, to which the splint is applied, generated based on the outer surface.

2. The method of claim 1, wherein:
the step of setting the outer surface includes:
generating a crop image including the outline and the inner surface set within the outline;
generating a smoothing image by minimizing a surface area of the crop image;
flattening the smoothing image to generate a flattened image;
dividing a region of the flattened image and changing a morphing direction according to the divided region to generate a morphing image for the flattened image; and
adjusting the morphing image based on a predetermined thickness.

3. The method of claim 2, wherein:
the generating of the smoothing image includes
performing a Laplacian-smoothing operation on the crop image.

4. The method of claim 2, wherein:
the generating of the flattened image includes
performing a convex hull operation on the smoothing image.

5. The method of claim 2, wherein:
the step of setting the outer surface further includes generating an offset image by processing an outer surface offset in a buccal direction and a lingual direction on the flattened image, and
the outer surface offset becomes thinner as it approaches the outline.

6. The method of claim 5, wherein:
the generating of the morphing image includes:
setting a plurality of interpolation points according to arrangement on the offset image; and
moving the plurality of interpolation points corresponding to an anterior region in a first direction, and moving the plurality of interpolation points corresponding to a post region in a second direction different from the first direction.

7. The method of claim 6, wherein:
the second direction is parallel to the occlusal plane and a normal vector direction.

8. The method of claim 6, wherein:
the generating of the morphing image includes
performing radial basis function (RBF) interpolation based on the plurality of moved interpolation points.

9. The method of claim 6, wherein:
the generating of the morphing image includes generating a plurality of image groups that are divided from each other in the offset image, and
the plurality of interpolation points are arranged to correspond to each of the plurality of image groups.

10. The method of claim 2, wherein:
the adjusting of the morphing image based on the predetermined thickness includes
comparing the occlusal thickness with the predetermined thickness to reduce an occlusal thickness of at least a portion of the morphing image.

11. An electronic apparatus, comprising:
a user interface device;
a processor; and
a memory configured to store instructions executable by the processor,
wherein the processor is configured to execute the instructions to:
receive at least one intraoral image;
align the at least one intraoral image to an occlusal plane;
set an inner surface of a splint based on the at least one aligned intraoral image;
set an outline of the splint based on the at least one intraoral image;
set an outer surface of the splint based on the outline; and
display the at least one intraoral image, to which the splint is applied, generated based on the outer surface.

12. The electronic apparatus of claim 11, wherein:
the setting operation for the outer surface includes:
generating a crop image including the outline and the inner surface set within the outline;
generating a smoothing image by minimizing a surface area of the crop image;
flattening the smoothing image to generate a flattened image;
dividing a region of the flattened image and changing a morphing direction according to the divided region to generate a morphing image for the flattened image; and
adjusting the morphing image based on the predetermined thickness.

13. The electronic apparatus of claim 12, wherein:
the generating of the smoothing image includes
performing a Laplacian-smoothing operation on the crop image.

14. The electronic apparatus of claim 12, wherein:
the generating of the flattened image includes
performing a convex hull operation on the smoothing image.

15. The electronic apparatus of claim 12, wherein:
the setting operation for the outer surface further includes generating an offset image by processing an outer surface offset in a buccal direction and a lingual direction on the flattened image, and
the outer surface offset becomes thinner as it approaches the outline.

16. The electronic apparatus of claim 15, wherein:
the generating of the morphing image includes;
setting a plurality of interpolation points according to arrangement on the offset image; and
moving the plurality of interpolation points corresponding to an anterior region in a first direction, and moving the plurality of interpolation points corresponding to a post region in a second direction different from the first direction.

17. The electronic apparatus of claim 16, wherein:
the generating of the morphing image includes
performing radial basis function (RBF) interpolation based on the plurality of moved interpolation points.

18. The electronic apparatus of claim 16, wherein:
the generating of the morphing image includes generating a plurality of image groups that are divided from each other in the offset image, and
the plurality of interpolation points are arranged to correspond to each of the plurality of image groups.

19. The electronic apparatus of claim 12, wherein:
the adjusting of the morphing image based on the predetermined thickness includes
comparing the occlusal thickness with the predetermined thickness to reduce an occlusal thickness of at least a portion of the morphing image.

20. A computer readable storage medium including computer readable instructions,
wherein the instructions cause the computer to:
receive at least one intraoral image;
align the at least one intraoral image to an occlusal plane;
set an inner surface of a splint based on the at least one aligned intraoral image;
set an outline of the splint based on the at least one intraoral image;
set an outer surface of the splint based on the outline; and
display the at least one intraoral image, to which the splint is applied, generated based on the outer surface.
